# EUROPÄISCHE PATENTSCHRIFT

(11) **EP 0 649 428 B1**
(45) Veröffentlichungstag und Bekanntmachung des Hinweises auf die Patenterteilung: **10.11.1999**
(21) Anmeldenummer: 94909281.1
(22) Anmeldetag: 28.02.1994
(51) Int. Cl.: C07H 1/08

(54) **VERFAHREN ZUR HERSTELLUNG DES NATRIUMSALZES DER DNS UND VORRICHTUNG ZUR DURCHFÜHRUNG DES VERFAHRENS**
PROCESS AND DEVICE FOR PREPARING DNA SODIUM SALT
PROCEDE ET DISPOSITIF DE PREPARATION DU SEL DE SODIUM D'ADN

(30) Priorität: 29.04.1993 RU 3018605
(43) Veröffentlichungstag der Anmeldung: 26.04.1995
(73) Patentinhaber: EFTAG ENTSTAUBUNGS- UND FÖRDERTECHNIK AG, CH-2500 Biel 3 (CH); WEINBERG, Juri Petrowitsch, Moscow, 109172 (RU); KAPLINA, Elli Nikolaevna, Moscow, 109172 (RU); KAPLIN, Walerij Jurjewitsch, Moscow, 109172 (RU); LADIGIN, Alexander Jewgenjewitsch, Moscow, 125206 (RU)
(72) Erfinder: WEINBERG, Juri Petrowitsch, Moscow, 109172 (RU); KAPLINA, Elli Nikolajewna, Moscow, 109172 (RU); KAPLIN, Walerij Jurjewitsch, Moscow, 109172 (RU); LADIGIN, Alexander Jewgenjewitsch, Moscow, 125206 (RU)
(74) Vertreter: Spalthoff, Adolf, Dipl.-Ing.
(86) Internationale Anmeldenummer: IB9400054
(87) Internationale Veröffentlichungsnummer: WO9425473

(56) Entgegenhaltungen:
- WO-A-93/03150
- DE-B- 1 142 365
- FR-M- 2 147
- US-A- 5 155 018
- CHEMICAL PATENTS INDEX, DOCUMENTATION ABSTRACTS JOURNAL Section Ch, Week 9415, Derwent Publications Ltd., London, GB; Class B, AN 124657 & SU,A,2 004 972 (VAINBERG YU P) 30. Dezember 1993
- CHEMICAL PATENTS INDEX, DOCUMENTATION ABSTRACTS JOURNAL Section Ch, Week 9301, Derwent Publications Ltd., London, GB; Class B04, AN 007157 & SU,A,1 705 301 (BIOORGANIC CHEM. INST.) 15. Januar 1992
- CENTRAL PATENTS INDEX, BASIC ABSTRACTS JOURNAL Section Ch, Week 8007, Derwent Publications Ltd., London, GB; Class B04, AN 12004C & JP,A,55 002 635 (T.IWAI) 10. Januar 1980
- DNA, Bd.8, Nr.10, Dezember 1989, USA Seiten 697 - 701 H.I.ELSNER & E.B.LINDBLAD 'Ultrasonic degradation of DNA'
- ANALYTICAL BIOCHEMISTRY, Bd.129, Nr.1, 15. Februar 1983 Seiten 216 - 223 P.L.DEININGER 'Random subcloning of sonicated DNA: application to shotgun DNA sequence analysis'
- PROCEEDINGS OF THE NATIONAL ACADEMY OF SCIENCES OF USA, Bd.76, Nr.2, Februar 1979, WASHINGTON US Seiten 615 - 619 B.VOGELSTEIN & D.GILLESPIE 'Preparative and analytical purification of DNA from agarose'

## Beschreibung

Die Erfindung bezieht sich auf ein Verfahren zur Herstellung des Natriumsalzes der Desoxyribonucleinsäure (DNS) aus tierischem Rohmaterial, bei dem das tierische Rohmaterial zerkleinert und in Citratsalzlösung homogenisiert wird, das homogenisierte Material bei erhöhter Temperatur mit Detergentien und konzentrierter NaCl-Lösung bearbeitet wird, das reagierte Material abgekühlt wird, das abgekühlte Material mit Kieselgur vermischt wird, die Phasen durch Filtrieren voneinander getrennt werden und das Endprodukt mit Äthylalkohol ausgefällt wird. Des weiteren bezieht sich die Erfindung auf den Komplex eines Natriumsalzes der DNS, eine ein derartiges Natriumsalz enthaltende pharmazeutische Verbindung, eine den genannten Komplex enthaltende pharmazeutische Verbindung sowie auf eine Vorrichtung zur Herstellung des genannten Natriumsalzes.

Die Erfindung gehört auf das Gebiet der Technologie zur Herstellung biologisch aktiver Substanzen; sie kann in der Biotechnologie, in der chemisch-pharmazeutischen Industrie sowie an deren Produkten in der Human- und Tiermedizin und in der Kosmetik eingesetzt werden.

Bei einem aus dem russischen Erfinderzertifikat 487 897 bekannten Verfahren zur Herstellung von DNS aus tierischem Rohmaterial wird Störfischmilch, die als tierisches Rohmaterial dient, zerkleinert und danach in einer Citratsalzlösung homogenisiert. Daraufhin erfolgt eine Bearbeitung des reagierten Materials mit Detergentien und konzentrierter NaCl-Lösung, und zwar bei einer Temperatur zwischen 60 und 70 Grad C. Nach einer Abkühlung wird das Material mit Kieselgur vermischt, woraufhin die DNS mittels einer Citratsalzlösung eluiert wird. Das Eluat wird durch Filtrieren abgetrennt, woraufhin das Endprodukt mit Äthyl ausgefällt wird.

Nachteilig an dem vorstehend beschriebenen Verfahren ist die Tatsache, daß der Austrag des Endproduktes mit 20 % der im tierischen Rohmaterial vorhandenen Gesamtmenge vergleichsweise gering ist. Die so gewonnene DNS ist nur nach einer zusätzlichen Behandlung des erzielten Endprodukts zwecks begrenzter und kontrollierter Fragmentierung der Makromoleküle möglich, wodurch einerseits die für hochmolekulare DNS-Formen charakteristischen Eigenschaften, wie Toxizität, Mutagenität und Kanzerogenität eliminiert werden und andererseits die Viskosität der Lösung verringert wird. Hierbei wird häufig eine wesentliche Degradierung der nativen 2-Strang-Struktur der DNS beobachtet, als deren Folge die DNS einige wichtige biologische Eigenschaften verliert.

Insgesamt ist festzustellen, daß eine industrielle Herstellungsweise der DNS, zu der eine Zerkleinerung der DNS im Endstadium des Verfahrens gehört, aufgrund der technologischen Schwierigkeiten bei Trennung, Lösung und Weiterverarbeitung der stark viskosen Produkte praktisch unmöglich ist.

Der Erfindung liegt die Aufgabe zugrunde, ein Verfahren zur Herstellung des Natriumsalzes der DNS aus tierischem Rohmaterial zur Verfügung zu stellen, welches im industriellen Maßstab einsetzbar ist und das darüber hinaus einen hohen Austrag der im tierischen Rohmaterial vorhandenen DNS-Gesamtmenge sichert, wobei zugleich eine begrenzte Verringerung des Molekulargewichts unter Beibehaltung der nativen Struktur und der hohen Reinheit der DNS erreicht werden soll.

Diese Aufgabe wird erfindungsgemäß durch das eingangs geschilderte Verfahren gelöst, wobei das reagierte Material vor der Vermischung mit Kieselgur 10 bis 80 Minuten mit Ultraschall beaufschlagt wird, wobei das Material während dieser Ultraschallbeaufschlagung so geschichtet wird, daß es bei vorgegebenen Bedingungen und Temperaturen gleichmäßig und vollständig mit Ultraschall beaufschlagbar ist, wobei das Mischungsverhältnis zwischen Kieselgur und dem ultraschallbehandelten Material 1 : 5 bis 25 (Gew. in mg / Vol. in ml) beträgt, wobei die durch Filtrieren abgetrennte flüssige Phase durch Druckfiltration konzentriert wird und das ausgefällte Natriumsalz der DNS bei 20 bis 60 Grad C getrocknet wird.

Mit dem erfindungsgemäßen Verfahren können biologisch aktive Materialien isoliert, gereinigt und genutzt werden, mittels denen verschiedene Arten therapeutischer und kosmetischer Mittel herstellbar sind, die bei der Therapie vieler Erkrankungen sehr effektiv sind. Derartige das Natriumsalz der DNS enthaltende Mittel haben eine hohe biologische Aktivität und haben keine negative Wirkung auf die Mutagenese oder Kanzerogenese. Derartige Mittel sollen reparative und regenerative Prozesse in beschädigten Organen und Geweben stimulieren und das Immunsystem von Lebewesen aktivieren. Das Natriumsalz der nativen DNS kann zur Therapie der problematischen Folgen von Strahlen- und Chemotherapie, bei Bestrahlung, bei Befall der Schleimhaut im Mund und im Magen-Darm-Trakt, bei Gefäßerkrankungen, bei Diabetes mellitus, bei Verbrennungen, die durch Strahlen, thermisch oder chemisch hervorgerufen wurden, bei Wundinfektionen etc. und bei viralen Erkrankungen, die durch DNS- oder RNS-Viren hervorgerufen wurden, eingesetzt werden. Entsprechende Arzneimittel können parenteral, rektal, intranosal oder kutan verabreicht werden. Außerdem kann das Natriumsalz der nativen DNS in kosmetischen Mitteln eingesetzt werden, wobei es insbesondere zur Verlangsamung oder zur Vorbeugung der Hautalterung genutzt werden kann.

Bei Durchführung des erfindungsgemäßen Verfahrens wird das Natriumsalz der DNS aus tierischem Rohmaterial gewonnen. Durch die Ultraschallbearbeitung des Materials, die vorzugsweise mit einer Frequenz von 8 bis 35 kHz und einer Intensität von 0,2 bis 2,0 W/cm2 erfolgt, wird zum einen eine begrenzte und kontrollierte Fragmentierung der hochmolekularen DNS erreicht. Des weiteren ergeben sich Strukturveränderungen des reagierten Materials, mittels denen die weitere Reinigung erleichtert und die Qualität verbessert wird. Durch die gleichmäßige Schichtung des Materials wird eine gleichmäßige und vollständige Beaufschlagung erzielt, die eine Homogenität des ultraschallbearbeiteten Materials zur Folge hat.

Durch die Auswahl eines optimalen Mischungsverhältnisses zwischen Kieselgur und dem reagierten und ultraschallbehandelten Material werden die Leistung, der Austrag und die Reinigungsqualität des erfindungsgemäßen Verfahrens wesentlich beeinflußt. Eine Abweichung des Mischungsverhältnisses von dem optimalen Verhältnis führt zu einer Verlangsamung der Reaktionsgeschwindigkeit. Aufgrund der erfindungsgemäßen Konzentration der flüssigen Phase durch Druckfiltration wird gewährleistet, daß die DNS vollständig als Sediment ausfällt; was sich auf den Austrag des erfindungsgemäßen Verfahrens positiv auswirkt. Die Austrocknung des Natriumsalzes der DNS bei 20 bis 60 Grad C schützt das Endprodukt vor den möglichen negativen Einwirkungen beim Liophylisieren. Aufgrund der Verringerung des Molekulargewichtes und dem höheren Reinheitsgrad, welche mit dem erfindungsgemäßen Verfahren erreicht werden, kann dieser Verfahrensschritt überhaupt erst eingesetzt werden.

Vorteilhaft erfolgt die Druckfiltration mit Membranen, die eine Porengröße von 0,40 bis 1,00 Mkm aufweisen.

Als Salz der Citratsalzlösung kann zweckmäßigerweise ein Salz gehören, das zu derjenigen Gruppe gehört, die Natriumchlorid, Natriumsulfat, Natriumphosphat und Natriumphenylphosphat einschließt, oder zu derjenigen Gruppe, die Natriumcitrat, Natriumacetat oder das Natriumsalz der Äthylendiamintetraacetatsäure einschließt.

Das Natriumsalz der DNS wird in pharmazeutischen Verbindungen eingesetzt, die aus einer aktiven Komponente und einem oder mehreren pharmazeutisch akzeptablen Transmitter bzw. Transmittern bestehen. Die pharmazeutischen Verbindungen bzw. Präparate können mit verschiedenen Konzentrationen des Natriumsalzes der nativen DNS zusammengestellt werden. Es werden jedoch drei charakteristische Konzentrationen hergestellt und bewertet. Diese Verbindungen bzw. Präparate haben folgende Parameter und Gewichtsverhältnisse:
- sterile 1,5%ige Lösung des Natriumsalzes des DNS in 0,1%iger Wasserlösung des NaCl;
- sterile 0,5%ige Lösung des Natriumsalzes der DNS in 0,9%iger Wasserlösung des NaCl; und
- sterile 0,2%ige Lösung des Natriumsalzes der DNS in 0,1%iger Wasserlösung des NaCl.

Die Konzentration des Natriumsalzes der DNS in pharmazeutischen Präparaten kann bis zu 4 Gew.-% betragen, wobei aber bei einer derart hohen Konzentration die Lösungen sehr zähflüssig werden und sich ähnlich einem Gel verhalten. Der optimale Konzentrationsbereich liegt zwischen 0,1 und 4 Gew.-%, ausgehend von dem Gesamtgewicht des Präparats.

Dies gilt für alle Formen pharmazeutischer Präparate. Für parenterale Anwendung sollten die Verbindungen bzw. Präparate 0,5 bis 2,0 Gew.-% des Natriumsalzes der DNS enthalten, für intranosale und äußerliche Anwendung 0,1 bis 0,5 Gew.-%.

Bei Bedarf können pharmazeutische Verbindungen und Präparate, die das gemäß der vorliegenden Erfindung hergestellte Natriumsalz der DNS enthalten, auch andere Ingredienzen beinhalten. Hierfür können sowohl inerte als auch aktive Ingredienzen eingesetzt werden, sofern ihre Wirkung dem Effekt der Hauptkomponente nicht zuwiderläuft. Derartige pharmazeutische Präparate auf der Basis der DNS können durch Injektion, Tropfen, rektal oder durch äußerliche Anwendung appliziert werden.

Das erfindungsgemäß hergestellte Natriumsalz der DNS kann in einem Komplex mit einer Reihe physiologisch aktiver Substanzen und Mikroelementen eingebunden werden, wodurch Substanzen entstehen, die eine ausgeprägte antivirale Wirkung haben, und zwar sowohl gegen RNS- als auch gegen DNS-Viren. Mit dem Natriumsalz der DNS können nur solche Substanzen und Mikroelemente kombiniert werden, die eine feste Verbindung nur mit einem DNS-Strang der DNS bilden, ohne sich auch an dem zweiten DNS-Strang anzulagern.

Es sind unterschiedliche chemotherapeutische Substanzen bekannt, die den Tod oder die Vermehrungsunfähigkeit von RNS- oder DNS-Viren verursachen, so z.B. das Präparat Idoxuridin, das aktiv auf DNS-haltige Viren einwirkt, u.a. auf den Herpes-Virus, oder das Präparat Asidothymidin (ALT), das aktiv auf RNS-haltige Viren einwirkt, u.a. auf HIV-Viren. Alle diese bekannten Präparate sind jedoch nicht ausreichend wirksam und darüber hinaus sehr toxisch.

Im Gegensatz dazu sind diejenigen Präparate, die das erfindungsgemäß hergestellte Natriumsalz der DNS enthalten, sehr stark wirksam, sowohl gegen DNS- als auch gegen RNS-Viren, so z.B. gegen HIV-Viren. Außerdem sind diese Präparate nicht toxisch und sie haben keine negativen Nebenwirkungen. Substanzen, die mit dem erfindungsgemäß hergestellten Natriumsalz der DNS einen antiviralen Komplex bilden, können Bromethyd, Ethylen, Imin sein, nicht jedoch Adriomyzin; sie können Fe, Co, Ni, nicht jedoch Ag sein.

In einer weiteren Ausführungsform der vorliegenden Erfindung können die das Natriumsalz der DNS enthaltenden Verbindungen in der Kosmetik eingesetzt werden. Derartige kosmetische Mittel beugen der Hautpigmentierung vor, sie erleichtern das Entfernen von Akne, Pickel und Narben, sie intensivieren die Heilung von Verbrennungen, Schnitt- und anderen Wunden. Außerdem können sie als Mittel zur Verhinderung der Hautalterung eingesetzt werden.

Die erfindungsgemäße Vorrichtung zur Herstellung des Natriumsalzes der DNS aus tierischem Rohmaterial hat einen Arbeitstisch, auf dem gefrorenes tierisches Rohmaterial aufgebaut und von Fetten und Verunreinigungen befreit wird, einen Fleischwolf mit einer elektrischen Antriebseinrichtung, in dem das gereinigte Rohmaterial zu Gewebematerial verkleinert wird, einen Homogenisator, in dem das Gewebematerial fein zerkleinert und homogenisiert wird, einen Kochreaktor, in dem das homogenisierte Material erwärmt wird, einen Kühlreaktor, in dem das Material nach der Wärmebehandlung im Kochreaktor abgekühlt wird, eine Ultraschallbehandlungseinrichtung, in der das im Kochreaktor abgekühlte Material mit Ultraschall beaufschlagt wird, eine Mischeinrichtung, in der das mit Ultraschall behandelte Material mit Kieselgur gemischt wird, eine Trenneinrichtung zur Trennung der festen und flüssigen Phase des mit Kieselgur gemischten Materials, eine Filtriereinrichtung, in der die abgetrennte flüssige Phase fein filtriert und konzentriert wird, einen Ausfällreaktor, in dem das Endprodukt ausgefällt wird, eine Separiereinrichtung, in der das ausgefällte Endprodukt separiert wird, und eine Trocknungseinrichtung, in der das separierte Endprodukt in Form des Natriumsalzes der DNS getrocknet wird.

Vorteilhaft weisen der Kochreaktor und der Kühlreaktor jeweils ein Niedriggeschwindigkeitsrührwerk, einen Wärmetauschermantel, einen Temperaturregler und eine Pneumolufteinrichtung auf.

Die Ultraschallbehandlungseinrichtung hat zweckmäßigerweise zumindest eine Wanne, an deren Boden ein magnetostriktiver Ultraschallgeber angeordnet ist, wobei der Spalt zwischen dem Boden bzw. den Seitenwänden der Wanne und dem magnetostriktiven Ultraschallgeber maximal 2 cm breit ist.

Des weiteren hat jede Wanne vorteilhaft eine Ablaßeinrichtung und eine als Rohrwärmetauscher ausgebildete Kühlanlage, die in der Wanne oberhalb des magnetostriktiven Ultraschallgebers angeordnet ist.

In der Mischeinrichtung ist zweckmäßigerweise ein Hochleistungsrührwerk vorgesehen.

Zur Erhöhung der Reinigungswirkung und zur Verbesserung der Konzentration hat die Filtriereinrichtung eine Feinfiltriereinrichtung zur Mikrofiltration der abgetrennten flüssigen Phase und eine Druckmembraneinrichtung zur Konzentration der mikrofiltrierten flüssigen Phase.

Mit der vorstehend geschilderten Vorrichtung zur Herstellung des Natriumsalzes der DNS kann das genannte Produkt im industriellen Maßstab mit einem hohen Reinheitsgrad und mit einer bestimmten Fragmentengröße unter Beibehaltung der nativen Struktur der DNS hergestellt werden.

Aus der FR-A 2 147 M ist es grundsätzlich bekannt, biologische Stoffe mittels Ultraschallverfahren zu behandeln.

Aus dem letzten Kapitel von DNA, 1989, Seiten 697 bis 701, ist es bekannt, mit Proteinen verbundene DNA mittels Enzym-, Säure- und Alkali-Hydrolyse zu bearbeiten bzw. weiterzuverarbeiten. Eine Ultraschallbehandlung wird dort im Zusammenhang mit der Degradierung von DNA beschrieben, wobei DNA-Fragmente zur Erzeugung von Weiterverarbeitungsprodukten erzielt werden.

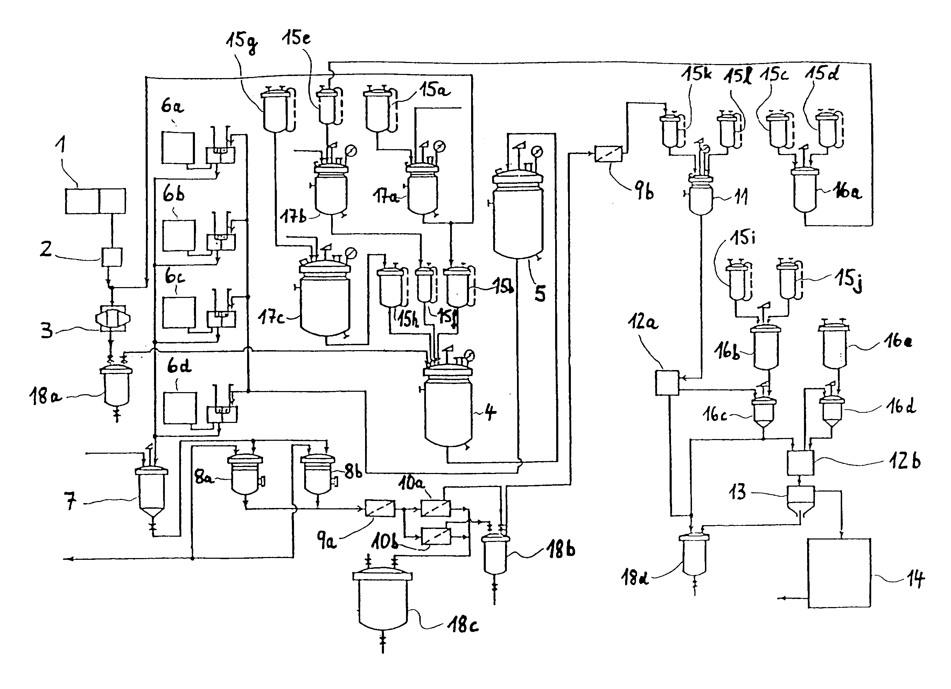

Im folgenden wird die Erfindung an Hand von Ausführungsbeispielen, teilweise unter Bezugnahme auf die Zeichnung, näher erläutert.

In der einzigen Figur ist eine Ausführungsform der erfindungsgemäßen Vorrichtung zur Herstellung des Natriumsalzes der DNS prinzipiell dargestellt.

Bei einer ersten Ausführungsform des erfindungsgemäßen Verfahrens wird 1 kg Störfischmilch in einem Fleischwolf zerkleinert und dann in 2 1 Citratsalzlösung (0,15 MNaCl und 0,15 M Citrat-Na homogenisiert; daraufhin wird das homogenisierte Material mit 3 1 erwärmter Citratsalzlösung in einen Reaktor eingebracht, wobei des weiteren 10 1 6%iger Natriumdodecylsulfatlösung in 45%igem Äthylalkohol zugesetzt werden. Das Reaktionsgemisch verbleibt bei einer Temperatur von 60 Grad C und unter ständigem Umrühren für 1,5 Stunden im Reaktor. Danach wird im gleichen Volumen 5M NaCl-Lösung zugeführt, wonach das im Reaktor befindliche Reaktionsgemisch unter Beibehaltung derselben Temperatur weitere 1,5 Stunden gerührt wird. Daraufhin wird die Reaktionsmasse auf Zimmertemperatur abgekühlt.

Die abgekühlte Reaktionsmasse wird in Wannen eingegossen, wobei ihre Schichtdicke ca. 15 mm betragen sollte; die derart geschichtete Reaktionsmasse wird in den Wannen mit Ultraschall der Frequenz 8 kHz und der Intensität 2 W/cm2 für 80 Minuten behandelt. Die so bearbeitete Reaktionsmasse wird im Mischungsverhältnis von 5 : 1 mit Kieselgurpulver vermischt. Danach wird die flüssige Phase der mit dem Kieselgurpulver vermischten Reaktionsmasse auf einem Nutschenfilter abgetrennt.

Nach einer zusätzlichen Mikrofiltrierung wird das Filtrat auf einer Druckmembran mit einer Porengröße von 0,45 Mkm eingedickt.

Derart erhält man 8 1 Konzentrat, welches 7,5 Gew.-% DNS als Natriumsalz enthält. Das Endprodukt erhält man mittels Ausfällung in Äthylalkohol. Der Ausfall bzw. das Sediment wird durch Zentrifugierung abgetrennt und bei 20 bis 40 Grad C für 48 Stunden getrocknet.

Hierdurch erhält man 60 g des Natriumsalzes der DNS mit folgenden Charakteristika:
Molekulargewicht 400 +/- 100 kD Eiweißgehalt maximal 1 %, RMS-Gehalt max. 2 %, Polysaccaridengehalt max. 2 %, Feuchte 17 %, Hyperchromeffekt 44 %. Das Endprodukt ist ein weißes, amorphes Pulver.

Bei dem gemäß der vorstehend geschilderten Ausführungsform des erfindungsgemäßen Verfahrens hergestellten Natriumsalz der DNS ergeben sich eine kontrollierbare Fragmentgröße, eine Stabilität der sekundären Struktur der Makromoleküle und ein hoher Reinheitsgrad. Hierdurch ist der Einsatz des erfindungsgemäß hergestellten Natriumsalz der DNS in der Forschung und in der Gentechnik möglich.

Eine begrenzte und kontrollierte Verringerung des Molekulargewichts ermöglicht den Einsatz des Natriumsalzes der DNS in Human- und Veterinärmedizin sowie in der kosmetischen Industrie.

In der einzigen Figur ist eine erfindungsgemäße Vorrichtung zur Herstellung des Natriumsalzes der DNS aus tierischem Rohmaterial dargestellt. Zur Vorrichtung gehören ein Arbeitstisch 1, ein Fleischwolf 2 mit Elektroantrieb, ein Gewebefeinzerkleinerer bzw. Homogenisator 3, ein Kochreaktor 4, ein Kühlreaktor 5, eine Ultraschallbehandlungseinrichtung 6a, 6b, 6c, 6d, eine Mischeinrichtung 7, eine Trenneinrichtung 8a, 8b, die beispielsweise durch Nutschenfilter ausgebildet sein kann, eine Mikro- bzw. Feinfiltriereinrichtung 9a, 9b, eine Druckmembraneinrichtung 10a, 10b zur Eindickung bzw. Konzentration der flüssigen Phase, ein Ausfällreaktor 11, eine Separiereinrichtung 12a, 12b zum Abtrennen des ausgefällten Sediments, deren Einzelelemente als Zentrifugen ausgebildet sein können, ein Schotttrichter 13, und eine Trocknungseinrichtung 14 in Form eines Trockenschranks.

Des weiteren enthält die erfindungsgemäße Vorrichtung zur Herstellung des Natriumsalzes der DNS Meßbehälter 15a, 15b, 15c, 15d, 15e, 15f, 15g, 15h, 15i, 15j, 15k, 151 zur Dosierung von Flüssigkeiten, Mischbehälter 16a, 16b, 16c, 16d, 16e, die zur Herstellung der Lösungen und Suspensionen bei Zimmertemperatur dienen, Reaktorbehälter 17a, 17b, 17c, die zur Herstellung der Lösungen und Gemische bei sich von der Zimmertemperatur unterscheidenden Temperaturen dienen, und Sammelbehälter 18a, 18b, 18c, 18d, die zur Aufnahme von Zwischenprodukten und Verfahrensabfällen dienen.

Die Mischeinrichtung 7 zur Mischung des ultraschallbehandelten Reaktionsgemisches mit Kieselgur hat ein schnellrotierendes Hochleistungsrührwerk, welches z.B. mit einer Drehzahl von 8000 U/min arbeitet. Die Mischbehälter 16a, 16b, 16c, 16d, 16e und die Sammelbehälter 18a, 18b, 18c, 18d, die Alkohol oder Alkohol lösungen enthalten, sind zusätzlich hermetisch isoliert.

Die Reaktorbehälter 17a, 17b, 17c sind hermetisch isoliert und weisen Mischeinrichtungen, eine Auskleidung zur Isolierung gegen Wärme oder Kälte, temperaturregelnde Einrichtungen und Drucklufteinrichtungen auf.

Der Kochreaktor 4 hat ein Niedriggeschwindigkeitsrührwerk, das z.B. als Ankermischer ausgebildet ist.

Die Elemente der erfindungsgemäßen Vorrichtung zur Herstellung von Natriumsalz der DNS sind mittels Rohrleitungen miteinander verbunden.

Die Ultraschallbehandlungseinrichtung 6a, 6b, 6c, 6d hat mehrere Wannen. An jedem Wannenboden ist ein magnetostriktiver Ultraschallgeber vorgesehen, wobei der Spalt zwischen dem magnetostriktiven Ultraschallgeber und den Seitenwänden der betreffenden Wanne max. 2 cm beträgt. Des weiteren weist jede Wanne eine Kühlanlage auf, die vorzugsweise als Rohrwärmetauscher ausgebildet ist. Diese Kühlanlage ist in der Wanne so angeordnet, daß zwischen ihr und dem magnetostriktiven Ultraschallgeber keine Berührung auftreten kann. Jede Wanne hat eine Öffnung zum Ablassen der Reaktionsmasse.

Im folgenden wird die Arbeitsweise der erfindungsgemäßen Vorrichtung zur Herstellung des Natriumsalzes der DNS beschrieben.

Frisch gefrorenes tierisches Rohmaterial in Form von Störfischmilch wird auf den Arbeitstisch 1 aufgegeben und dort aufgetaut; nach dem Auftrauen wird das tierische Rohmaterial von Fett, Filmen ud.dgl. gesäubert.

Das derart gesäuberte tierische Rohmaterial wird mit dem Fleischwolf 2 zerkleinert und danach im Homogenisator 3 mit Citratsalzlösung homogenisiert.

Das homogenisierte Material wird im Sammelbehälter 18a gesammelt und von dort aus in den Kochreaktor 4 weitergeleitet.

Abgewogenes Natriumcitrat und Natriumchlorid werden in den Reaktorbehälter 17a eingeführt und mit der erforderlichen Menge destillierten Wassers, das aus dem Meßbehälter 15a in den Reaktorbehälter 17a eingeleitet wird, vermischt.

Hierzu wird die Mischeinrichtung des Reaktorbehälters 17a eingeschaltet, wobei der Rührvorgang anhält, bis die eingegebenen Komponenten völlig aufgelöst sind. Die fertige Citratsalzlösung wird aus dem Reaktorbehälter 17a in den Meßbehälter 15b gedrückt.

Aus dem Meßbehälter 15b bzw. aus der Rohrleitung zwischen dem Reaktorbehälter 17a und dem Meßbehälter 15b wird die Lösung in den Kochreaktor 4 bzw. zum Homogenisator 3 geleitet.

Äthylalkohol aus dem Meßbehälter 15c und destilliertes Wasser aus dem Meßbehälter 15d fließen in den Mischbehälter 16a, der ein Rührwerk aufweist, wobei das Verhältnis zwischen Äthylalkohol und destilliertem Wasser so gewählt ist, daß im Mischbehälter 16a eine 45%ige Alkohollösung hergestellt wird.

Die 45%ige Alkohollösung wird aus dem Mischbehälter 16a in den Meßbehälter 15e gepumpt, aus dem heraus die Alkohollösung in den Reaktorbehälter 17b eingeleitet wird.

In den mit der 45%igen Alkohollösung gefüllten Reaktorbehälter 17b wird Dodezylsulfat aufgegeben, danach wird die Mischeinrichtung bzw. das Rührwerk des Reaktorbehälterrs 17b eingeschaltet und die Alkohollösung sowie das Dodezylsulfat bis zur völligen Auflösung gerührt.

Die so hergestellte Wasser-Alkohol-Lösung vom Natriumdodezylsulfat wird in den Meßbehälter 15f gepumpt, von wo aus sie in den Kochreaktor 4 eingeleitet wird.

In den Reaktorbehälter 17c wird eine abgewogene Menge Natriumchlorid aufgegeben. Des weiteren fließt aus dem Meßbehälter 15g destilliertes Wasser in den Reaktorbehälter 17c. Die Mischeinrichtung des Reaktors 17c wird eingeschaltet, bis das Natriumchlorid völlig im destillierten Wasser aufgelöst ist.

Die Natriumchloridlösung wird in den Meßbehälter 15h gedrückt, von dem aus die Natriumchloridlösung in den Kochreaktor 4 fließt.

In den Mischbehälter 16b fließen in dem erforderlichen Mischungsverhältnis destilliertes Wasser aus dem Meßbehälter 15i und 96%iger Äthylalkohol aus dem Meßbehälter 15j. Die entstehende 70%ige Äthylalkohollösung fließt aus dem Mischbehälter 16b in den Mischbehälter 16c.

In den Kochreaktor 4 werden nach dem Einbringen des Reaktionsgemisches aus dem Sammelbehälter 18a nacheinander die Citratsalzlösung aus dem Meßbehälter 15b, die Natriumdodezylsulfatlösung aus dem Meßbehälter 15f und 5 M Natriumchloridlösung aus dem Meßbehälter 15h eingeleitet. Die Bearbeitung des Reaktionsgemisches mit jeder der genannten Lösungen erfolgt bei ständigem Umrühren für eine bestimmte Zeit bei 60 bis 70 Grad C. Nach Ablauf der Kochzeit wird das Reaktionsgemisch in den Kühlreaktor 5 geleitet und dort auf Zimmertemperatur abgekühlt.

Das abgekühlte Reaktionsgemisch wird in die Wannen der Ultraschallbehandlungseinrichtung 6a, 6b, 6c, 6d eingebracht. Die Aufgabe erfolgt portionsweise, wobei in den Wannen die jeweils zweckmäßige Schichtdicke des Reaktionsgemisches eingehalten wird. Mittels der magnetostriktiven Ultraschallgeber wird das in die Wannen eingeführte abgekühlte Reaktionsgemisch bei den jeweils zweckmäßigen und erforderlichen Bedingungen bearbeitet.

Nach Beendigung der Ultraschallbehandlung wird das Reaktionsgemisch durch Ablaßeinrichtungen aus den Wannen der Ultraschallbehandlungseinrichtung 6a, 6b, 6c, 6d ausgelassen und in die Mischeinrichtung 7 eingebracht.

Gleichzeitig mit der Einbringung des ultraschallbearbeiteten Reaktionsgemisches wird in die Mischeinrichtung 7 die gewünschte Menge Kieselgurpulver eingegeben, wobei bei eingeschaltetem Hochleistungsrührwerk das ultraschallbehandelte Reaktionsgemisch und das Kieselgurpulver durchmischt werden. Die so entstehende Suspension wird portionsweise auf die Trenneinrichtung, die Nutschenfilter 8a, 8b aufweist, aufgegeben. Dort erfolgt eine Abtrennung der festen Phase der Suspension aus dem Reaktionsgemisch und dem Kieselgurpulver.

Die an den Nutschenfiltern 8a, 8b der Trenneinrichtung abgetrennte flüssige Phase wird auf eine Feinfiltriereinrichtung 9a aufgegeben und durchläuft dann die Druckmembraneinrichtung 10a, 10b, in der sie konzentriert bzw. eingedickt wird.

Das derart entstehende Konzentrat wird in den Sammelbehälter 18b eingeführt, wohingegen das Permeat in den Sammelbehälter 18c eingebracht wird, aus dem das Permeat zur Regeneration weitergeleitet wird.

Das Konzentrat aus dem Sammelbehälter 18b wird auf die Feinfiltriereinrichtung 9b aufgegeben, von der es in den Meßbehälter 15k geleitet wird.

Aus dem Meßbehälter 15k fließt das Konzentrat in den Ausfällreaktor 11, in den aus dem Meßbehälter 151 96%iger Äthylalkohol eingebracht wird. Im Ausfällreaktor 11 erfolgt die Ausfällung der DNS unter ständigem Betrieb des dem Ausfällreaktor 11 zugehörigen Rührwerks.

Nach dem Ausfällen fließt die Suspension aus dem Ausfällreaktor 11 zu einer Zentrifuge 12a der Separiereinrichtung.

In der Zentrifuge 12a durch Zentrifugieren abgetrenntes Sediment mit DNS wird in den Mischbehälter 16c eingeleitet, wo es mit dem 70%igen Äthylalkohol zusammen gerührt wird, der aus dem Mischbehälter 16b in den Mischbehälter 16c eingeleitet wird.

Die Suspension aus dem Mischbehälter 16c wird in eine Zentrifuge 12b der Separiereinrichtung eingeleitet, aus der heraus das Sediment in den Mischbehälter 16d eingeleitet und dort mit aus dem Mischbehälter 16e in den Mischbehälter 16d eingeleitetem 96%igem Alkohol homogenisiert.

Nach dem Waschprozeß im Mischbehälter 16d wird die Suspension erneut in die Zentrifuge 12b eingegeben, aus der das pastenförmige Sediment auf den Schotttrichter 13 aufgebracht wird, wo unter Unterdruck Restfeuchte abgetrennt wird.

Der Supernatant aus der Zentrifuge 12a, der Zentrifuge 12b und dem Schotttrichter 13 wird in den Sammelbehälter 18d eingeleitet und regeneriert.

Das trockene Pulver aus dem Schotttrichter 13 wird auf Paletten aufgetragen und im Trockenschrank 14 bis auf die gewünschte vorgegebene Feuchte getrocknet. Das getrocknete Pulver wird dann aufgeteilt und verpackt.

In weiterer Ausgestaltung der vorliegenden Erfindung wird zur Herstellung einer Injektionslösung, die 1,5 % Natriumsalz der DNS in 0,1%iger Natriumchloridlösung enthält, 1 g Natriumchlorid in 1 1 bidestilliertem apyrogenen Wasser bei Zimmertemperatur gelöst. Zu dieser Lösung wird 18,75 g Pulver des Natriumsalzes der DNS zugegeben; unter ständigem Umrühren und bei Einhaltung der Zimmertemperatur wurde das Pulver für 3 Stunden aufgelöst. Die entstehende Lösung wurde durch nachgeschaltete Druckmembranfilter mit Porendurchmessern von 0,8 µm, 0,4 µm und 0,22 µm filtriert. Das Filtrat hinter dem letzten Druckmembranfilter wird in sterile 10 ml-Fläschchen abgefüllt.

0,25%ige Lösung des Natriumsalzes der DNS in 0,1%iger Natriumchloridlösung zur äußerlichen Applikation wird in folgender Weise vorbereitet:

1 g Natriumchlorid wird in 1 1 bidestilliertes appyrogenes Wasser bei einer Temperatur von 25 Grad C gelöst. Dieser Lösung werden 3,125 g Pulver des Natriumsalzes der DNS hinzugefügt; das Pulver wird durch Umrühren während 1,5 Stunden bei Zimmertemperatur aufgelöst. Die entstehende Lösung wurde wie beim vorstehend geschilderten Verfahrensablauf durch nachgeschaltete Druckmembranfilter filtriert. Das Filtrat wird in Fläschchen abgefüllt, die verschlossen und markiert werden.

## Patentansprüche

1. Verfahren zur Herstellung des Natriumsalzes der Desoxyribonucleinsäure (DNS) aus tierischem Rohmaterial, bei dem das tierische Rohmaterial zerkleinert und in Citratsalzlösung homogenisiert wird, das homogenisierte Material bei erhöhter Temperatur mit Detergentien und konzentrierter Natriumchloridlösung bearbeitet wird, das reagierte Material abgekühlt wird, das abgekühlte Material mit Kieselgur vermischt wird, die Phasen durch Filtrieren voneinander getrennt werden und das Endprodukt mit Äthylalkohol ausgefällt wird, dadurch gekennzeichnet, daß das reagierte Material vor der Vermischung mit Kieselgur 10 bis 80 Minuten mit Ultraschall beaufschlagt wird, wobei das Material so geschichtet wird, daß es bei vorgegebenen Bedingungen und Temperaturen gleichmäßig und vollständig beaufschlagbar ist, daß das Mischungsverhältnis zwischen Kieselgur und dem ultraschallbehandelten Material 1 : 5 bis 25 (Gewicht in mg / Vol. in ml) beträgt, daß die durch Filtrieren abgetrennte flüssige Phase durch Druckfiltration konzentriert wird und daß das ausgefällte Natriumsalz der DNS bei 20 bis 60 Grad C getrocknet wird.

2. Verfahren nach Anspruch 1, bei dem das reagierte Material mit Ultraschall mit einer Frequenz von 8 bis 35 kHz und einer Intensität von 0,2 bis 2,0 W/cm2 beaufschlagt wird.

3. Verfahren nach Anspruch 1 oder 2, bei dem die Druckfiltration mit Druckmembranfiltern, die eine Porengröße von 0,40 bis 1,00 µm aufweisen, erfolgt.

4. Verfahren nach einem der Ansprüche 1 bis 3, bei dem das Salz der Citratsalzlösung zu der Gruppe gehört, die Natriumchlorid, Natriumsulfat, Natriumphosphat und Natriumphenylphosphat einschließt.

5. Verfahren nach einem der Ansprüche 1 bis 3, bei dem das Salz der Citratsalzlösung zu der Gruppe gehört, die Natriumcitrat, Natriumacetat und Natriumsalz der Äthylendiamintetraacetatsäure einschließt.

6. Komplex eines Natriumsalzes der DNS, das nach einem Verfahren gemäß einem der Ansprüche 1 bis 5 hergestellt ist, der physiologisch aktive Substanz und/oder Mikroelemente enthält, dadurch gekennzeichnet, daß die physiologisch aktive Substanz und/oder die Mikroelemente ausschließlich Verbindungen mit einem der beiden Stränge der DNS eingehen.

7. Pharmazeutische Verbindung, enthaltend eine therapeutische Menge des gemäß einem der Ansprüche 1 bis 5 hergestellten Natriumsalzes der DNS und einen therapeutisch akzeptablen Transmitter.

8. Pharmazeutische Verbindung, enthaltend den Komplex des Natriumsalzes der DNS gemäß Anspruch 6 und einen therapeutisch akzeptablen Transmitter.

9. Kosmetisches Mittel zur Hautbehandlung, enthaltend kosmetische Grundsubstanz und das gemäß einem der Ansprüche 1 bis 5 hergestellte Natriumsalz der DNS.

10. Vorrichtung zur Herstellung des Natriumsalzes der DNS aus tierischem Rohmaterial gemäß dem Verfahren nach einem Ansprüche 1 bis 5, mit einem Arbeitstisch (1), auf dem gefrorenes tierisches Rohmaterial auftau- und von Fetten und Verunreinigungen befreibar ist, einem Fleischwolf (2) mit einer elektrischen Antriebseinrichtung, in dem das gereinigte Rohmaterial zu Gewebematerial zerkleinerbar ist, einem Homogenisator (3), in dem das Gewebematerial feinzerkleiner- und homogenisierbar ist, einem Kochreaktor (4), in dem das homogenisierte Material erwärmbar ist, einem Kühlreaktor (5), in dem das Material nach der Wärmebehandlung im Kochreaktor (4) abkühlbar ist, einer Ultraschallbehandlungseinrichtung (6a, 6b, 6c, 6d), in der das im Kühlreaktor (5) abgekühlte Material mit Ultraschall beaufschlagbar ist, einer Mischeinrichtung (7), in der das mit Ultraschall behandelte Material mit Kieselgur mischbar ist, einer Trenneinrichtung (8a, 8b) zur Trennung der festen und flüssigen Phase des mit Kieselgur gemischten Materials, einer Filtriereinrichtung (9a, 9b, 10a, 10b), in der die abgetrennte flüssige Phase feinfiltrier- und konzentrierbar ist, einem Ausfällreaktor (11), in dem das Endprodukt ausfällbar ist, einer Separiereinrichtung (12a, 12b, 13), in der das ausgefällte Endprodukt separierbar ist, und einer Trocknungseinrichtung (14), in der das separierte Endprodukt in Form des Natriumsalzes der DNS trockenbar ist.

11. Vorrichtung nach Anspruch 10, bei der der Kochreaktor (4) und der Kühlreaktor (5) jeweils ein Niedriggeschwindigkeitsrührwerk, einen Wärmetauschermantel, einen Temperaturregler und eine Pneumolufteinrichtung aufweisen.

12. Vorrichtung nach Anspruch 10 oder 11, bei der die Ultraschallbehandlungseinrichtung (6a, 6b, 6c, 6d) zumindest eine Wanne aufweist, an deren Boden ein magnetostriktiver Ultraschallgeber angeordnet ist, wobei der Spalt zwischen dem Boden bzw. den Seitenwänden der Wanne und dem magnetostriktiven Ultraschallgeber max. 2 cm breit ist.

13. Vorrichtung nach Anspruch 12, bei der jede Wanne eine Ablaßeinrichtung und eine als Rohrwärmetauscher ausgebildete Kühlanlage aufweist, die in der Wanne oberhalb des magnetostriktiven Ultraschallgebers angeordnet ist.

14. Vorrichtung nach einem der Ansprüche 10 bis 13, bei der die Mischeinrichtung (7) ein Hochleistungsrührwerk aufweist.

15. Vorrichtung nach einem der Ansprüche 10 bis 14, bei der die Filtriereinrichtung (9a, 9b, 10a, 10b) eine Feinfiltriereinrichtung (9a, 9b) zur Mikrofiltration der abgetrennten flüssigen Phase und eine Druckmembraneinrichtung (10a, 10b) zur Konzentration der mikrofiltrierten flüssigen Phase aufweist.

## Claims

1. Process for the preparation of the sodium salt of deoxyribonucleic acid (DNA) from animal raw material, wherein the animal raw material is comminuted, and then homogenised in citrate salt solution, the homogenised material is processed at elevated temperature with detergents and concentrated sodium chloride solution, the reacted material is cooled, the cooled material is mixed with diatomaceous earth, the phases are separated from one another by filtration and the end product is precipitated with ethyl alcohol, characterised in that the reacted material, before being mixed with diatomaceous earth, is acted upon by ultrasound for from 10 to 80 minutes, the material being so layered that it can be acted upon uniformly and completely under predetermined conditions and temperatures, in that the mixing ratio between diatomaceous earth and the ultrasoundtreated material is 1 : 5 to 25 (weight in mg / volume in ml), in that the liquid phase separated by filtration is concentrated by pressure filtration, and in that the precipitated sodium salt of the DNA is dried at from 20 to 60 degrees C.

2. Process according to claim 1, wherein the reacted material is acted upon by ultrasound at a frequency of from 8 to 35 kHz and at an intensity of from 0.2 to 2.0 W/cm².

3. Process according to claim 1 or 2, wherein the pressure filtration is effected with pressure membrane filters having a pore size of from 0.40 to 1.00 µm.

4. Process according to any one of claims 1 to 3, wherein the salt of the citrate salt solution belongs to the group that includes sodium chloride, sodium sulphate, sodium phosphate and sodium phenyl phosphate.

5. Process according to any one of claims 1 to 3, wherein the salt of the citrate salt solution belongs to the group that includes sodium citrate, sodium acetate and the sodium salt of ethylenediaminetetraacetic acid.

6. Complex of a DNA sodium salt prepared by a process according to any one of claims 1 to 5, which complex contains physiologically active substance and/or microelements, characterised in that the physiologically active substance and/or the microelements enter exclusively compounds with one of the two strands of the DNA.

7. Pharmaceutical compound, containing a therapeutic amount of the DNA sodium salt prepared according to any one of claims 1 to 5 and a therapeutically acceptable transmitter.

8. Pharmaceutical compound containing the complex of the DNA sodium salt according to claim 6 and a therapeutically acceptable transmitter.

9. Cosmetic agent for skin treatment, containing cosmetic base substance and the DNA sodium salt prepared according to any one of claims 1 to 5.

10. Apparatus for the preparation of the sodium salt of DNA from animal raw material by the process according to any one of claims 1 to 5, having a working table (1) on which frozen animal raw material can be thawed and freed from fats and impurities, a meat-mincing machine (2) which has an electrical drive device and in which the purified raw material can be comminuted to form tissue material, a homogeniser (3) in which the tissue material can be finely comminuted and homogenised, a boiling reactor (4) in which the homogenised material is heatable, a cooling reactor (5) in which the material is coolable after the heat treatment in the boiling reactor (4), an ultrasound treatment device (6a, 6b, 6c, 6d) in which the material cooled in the cooling reactor (5) can be acted upon by ultrasound, a mixing device (7) in which the material treated with ultrasound is mixable with diatomaceous earth, a separating device (8a, 8b) for separating the solid and liquid phase of the material mixed with diatomaceous earth, a filtering device (9a, 9b, 10a, 10b) in which the separated liquid phase can be fine-filtered and concentrated, a precipitation reactor (11) in which the end product is precipitable, a separating device (12a, 12b, 13) in which the precipitated end product is separable, and a drying device (14) in which the separated end product is dryable in the form of the sodium salt of DNA.

11. Apparatus according to claim 10, wherein each of the boiling reactor (4) and the cooling reactor (5) has a low-speed agitator, a heat exchanger jacket, a temperature regulator and a pneumatic air device.

12. Apparatus according to claim 10 or 11, wherein the ultrasound treatment device (6a, 6b, 6c, 6d) has at least one tank on the base of which a magnetostrictive ultrasound transmitter is arranged, the gap between the base and the side walls of the tank and the magnetostrictive ultrasound transmitter being a maximum of 2 cm wide.

13. Apparatus according to claim 12, wherein each tank has a discharge device and a cooling system which is in the form of a tubular heat exchanger and which is arranged in the tank above the magnetostrictive ultrasound transmitter.

14. Apparatus according to any one of claims 10 to 13, wherein the mixing device (7) has a heavy-duty agitator.

15. Apparatus according to any one of claims 10 to 14, wherein the filtering device (9a, 9b, 10a, 10b) has a fine-filtering device (9a, 9b) for the microfiltration of the separated liquid phase and a pressure membrane device (10a, 10b) for concentrating the microfiltered liquid phase.

## Revendications

1. Procédé de préparation d'un sel de sodium d'acide désoxyribonucléique (ADN) à partir d'une matière première animale, dans lequel on coupe en petits morceaux cette matière première animale et on l'homogénéise dans une solution de sel et de citrate, on traite cette substance homogénéisée à température élevée avec des détergents et une solution concentrée de chlorure de sodium, on refroidit le produit de réaction, on mélange ce produit refroidi avec de la terre d'infusoires, on sépare les phases l'une de l'autre par filtration, et l'on fait précipiter le produit final avec de l'alcool éthylique,
ce procédé étant **caractérisé** en ce que, avant de mélanger avec de la terre d'infusoires le produit de réaction, on lui fait subir un traitement aux ultrasons durant 10 à 80 minutes, ce produit étant alors disposé en couches de telle sorte qu'il peut, dans les conditions et à la température prévues, être entièrement et uniformément traité aux ultrasons, en ce que la proportion dans laquelle on mélange la terre d'infusoires et le produit traité aux ultrasons vaut de 1/5 à 1/25 (en poids/volume, mg/ml), en ce que l'on concentre, par filtration sous pression, la phase liquide séparée par filtration, et en ce que l'on fait sécher, à une température de 20 à 60 °C, le précipité de sel de sodium d'ADN.

2. Procédé conforme à la revendication 1, dans lequel on traite le produit de réaction avec des ultrasons dont le fréquence vaut de 8 à 35 kHz et dont l'intensité vaut de 0,2 à 2,0 W/cm².

3. Procédé conforme à la revendication 1 ou 2, dans lequel on effectue la filtration sous pression à l'aide de membranes filtrantes dont les pores présentent une taille de 0,40 à 1,00 µm.

4. Procédé conforme à l'une des revendications 1 à 3, dans lequel le sel de la solution de sel et de citrate appartient à l'ensemble constitué par les chlorure de sodium, sulfate de sodium, phosphate de sodium et phénylphosphate de sodium.

5. Procédé conforme à l'une des revendications 1 à 3, dans lequel le sel de la solution de sel et de citrate appartient à l'ensemble constitué par les citrate de sodium, acétate de sodium et éthylènediaminetétraacétate de sodium.

6. Complexe d'un sel de sodium d'ADN préparé selon un procédé conforme à l'une des revendications 1 à 5, qui contient des substances physiologiquement actives et/ou des oligo-éléments, **caractérisé** en ce que les substances physiologiquement actives et/ou les oligo-éléments n'entrent en combinaison qu'avec un seul des deux brins de l'ADN, exclusivement.

7. Composition pharmaceutique qui contient une quantité thérapeutique d'un sel de sodium d'ADN, préparé selon un procédé conforme à l'une des revendications 1 à 5, et un véhicule admissible du point de vue thérapeutique.

8. Composition pharmaceutique qui contient un complexe d'un sel de sodium d'ADN, conforme à la revendication 6, et un véhicule admissible du point de vue thérapeutique.

9. Agent cosmétique pour traitement de la peau, qui contient une base cosmétique et un sel de sodium d'ADN préparé selon un procédé conforme à l'une des revendications 1 à 5.

10. Installation servant à préparer un sel de sodium d'ADN à partir d'une matière première animale, selon un procédé conforme à l'une des revendications 1 à 5, et comportant une table de travail (1) sur laquelle on peut faire décongeler la matière première animale congelée et la débarrasser des graisses et impuretés, un hachoir (2) muni d'un moteur électrique, avec lequel on peut couper en petits morceaux de matériau tissulaire la matière première nettoyée, un homogénéisent (3) dans lequel on peut réduire en morceaux encore plus fins le matériau tissulaire et l'homogénéiser, un réacteur-chaudière (4) dans lequel on peut faire chauffer la substance homogénéisée, un réacteur-refroidisseur (5) dans lequel on peut faire refroidir cette substance après le traitement thermique effectué dans le réacteur-chaudière (4), un dispositif (6a, 6b, 6c, 6d) de traitement aux ultrasons, dans lequel on peut traiter avec des ultrasons la substance refroidie dans le réacteur-refroidisseur (5), un mélangeur (7) dans lequel on peut mélanger avec de la terre d'infusoires la substance traitée aux ultrasons, un séparateur (8a, 8b) qui permet de séparer les phases solide et liquide du mélange de substance et de terre d'infusoires, un dispositif de filtration (9a, 9b, 10a, 10b) dans lequel on peut filtrer plus finement la phase liquide séparée et la concentrer, un réacteur à précipitation (11) dans lequel on peut faire précipiter le produit final, un autre séparateur (12a, 12b, 13) dans lequel on peut séparer le produit final précipité, et un séchoir (14) dans lequel on peut faire sécher le produit final séparé, c'est-à-dire le sel de sodium d'ADN.

11. Installation conforme à la revendication 10, dans laquelle le réacteur-chaudière (4) et le réacteur-refroidisseur (5) comportent chacun un agitateur à faible vitesse, une chemise à échange de chaleur, un dispositif thermorégulateur, et un dispositif pneumatique à air.

12. Installation conforme à la revendication 10 ou 11, dans laquelle le dispositif (6a, 6b, 6c, 6d) de traitement aux ultrasons comporte au moins une cuve au fond de laquelle est disposé un générateur d'ultrasons à magnétostriction, avec un espace libre d'au plus 2 cm de large entre ce générateur d'ultrasons à magnétostriction et le fond ou la paroi latérale de la cuve.

13. Installation conforme à la revendication 12, dans laquelle chaque cuve comporte un dispositif de vidange et un réfrigérant qui présente la structure d'un échangeur thermique tubulaire et qui est disposé, à l'intérieur de la cuve, au-dessus du générateur d'ultrasons à magnétostriction.

14. Installation conforme à l'une des revendications 10 à 13, dans laquelle le mélangeur (7) comporte un agitateur mécanique à haut rendement.

15. Installation conforme à l'une des revendications 10 à 14, dans laquelle le dispositif de filtration (9a, 9b, 10a, 10b) comporte un dispositif de filtration fine (9a, 9b), pour soumettre à une microfiltration la phase liquide séparée, et un dispositif à membrane de filtration sous pression (10a, 10b), pour concentrer la phase liquide après cette microfiltration.
